# EUROPEAN PATENT SPECIFICATION

(11) **EP 0 386 649 B1**
(45) Date of publication and mention of the grant of the patent: **04.12.1996**
(21) Application number: 90104067.5
(22) Date of filing: 02.03.1990
(51) Int. Cl.: A61B 10/00

(54) **Disposable probe for the determination of vaginal PH and other indices**
Einwegsonde zur Bestimmung des vaginalen PH's und anderer Bestimmungsgrössen
Sonde jetable pour la détermination du PH vaginal et d'autres indices

(30) Priority: 07.03.1989 IT 2068889 U
(43) Date of publication of application: 12.09.1990
(73) Proprietor: Nucci, Prospero, I-20125 Milan (IT)
(72) Inventor: Nucci, Prospero, I-20125 Milan (IT)
(74) Representative: Gervasi, Gemma, Dr.

(56) References cited:
- US-A- 4 311 140
- US-A- 4 340 066
- US-A- 4 633 886
- US-A- 4 784 158

## Description

### Background of the invention

The present invention refers to a disposable probe for the determination of the vaginal pH and other indices which presents peculiar characteristics and advantages if compared with the tools commonly used for these purposes.

The vaginal pH is a very important index both of physiological conditions and of pathological states and therefore it should be routinary determined during obstetric-gynaecological examinations.

The determination of the vaginal pH allows :
1) A specific diagnosis for various kinds of infections as for example Candida albicans vaginitis, Trichomonas vaginitis, Garderenella vaginitis, mycotic infections, ecc.
2) A precocious diagnosis of other pathologies of the genito-urinary tract.
3) The determination of the more fertile days.
4) To acquire indications about possible local and systemic alterations.

The vaginal pH, in normal conditions, is comprised between 3.5 and 4.5 but tends to increase when the conditions are alterated because of a pathology, local or not, after the administration of medicaments or because of paraphysiological conditions.

The use of vaginal pH as diagnostic tool is however strongly limited by the lack of appropriate instruments for the determination by routine.

The commonly used instruments are the vaginal electrical or electronic pHmeters, or the paper pH-indicators.

The vaginal pHmeters are very expensive apparatuses while the use of paper pH-indicators by direct contact with the vaginal mucosa is unadvisable since no scientific data showing the absence of a cancerigenic factor of the indicators are at present available.

A paper pH-indicator as above is known, for example, from US-A-4,784,158 which describes a vaginal testing applicator comprising a pipe with a probe movable therein through a hole from a retracted position to an extended one and vice-versa: a pH-indicator (litmus or pH-paper), secured to one end of the probe, is arranged for contacting the vaginal mucosa when the probe is in its extended position.

In addition, on the above known device the vaginal liquid enters (or can enter) into the cavity of the pipe, reacting with the pH-indicator, during the introduction phase of said device into the body, while for a correct determination of the vaginal pH the pH-indicator must react with the vaginal liquid only when the probe is near to the posterior fornix of the vagina: no reliable vaginal pH test can therefore be made through the above known device.

A different type of medical apparatus used as a diagnostic tool in the obstetric-gynaecological field is known from US-A-4,311,140, which refers to a vacuum curet.

We have now found an unexpensive handy and reliable probe, which allows the determination of vaginal pH by routine, overcoming all difficulties and disadvantages of the prior art.

Such probe consists of a pipe with closed head and is characterized in that an oval hole is provided in the wall of said pipe near to said head and in that one or more pH indicators or other indicators are applied on the internal surface of said head and/or on the internal cylindrical surface of the pipe.

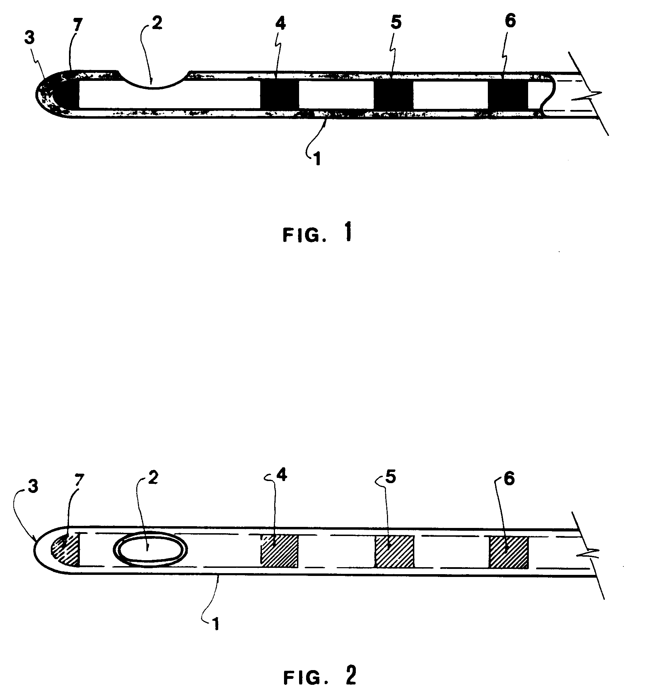

The characteristics and advantages of the probe according to the invention will be more evident from the following detailed description, referring to the enclosed explanatory Figures 1 and 2.

### Brief description of drawings

In the annexed drawings:
Figure 1 shows a side view of the probe according to the present invention and
Figure 2 shows a front view of the same probe of Figure 1.

### Description of the preferred embodiments

Referring to the reference numerals of the above said Figures, the probe, according to the invention, consists of a small pipe 1 presenting in the wall, near to the head, an oval hole 2 through which the vaginal secretion is collected.

According to the front view, the rim of such hole is inclined from up to down, from outside to inside, in order to permit an easier collection of the local secretion.

The major diameter of the hole is comprised between 8 and 12 mm.

The head 3 of the probe is oval, thoroughly smooth and has an increased thickness in respect of the wall of the pipe 1. On the internal surface of the pipe and on the internal surface of the head are applied one or more indicators, for example in positions 4, 5 and 6 opportunely spaced, and in position 7.

Such indicators are those used for the pH determination and particularly for pH values from 3,5 to 8.

In the above said positions other indicators can also be applied (colorimetric, physical, etc.) which can be used for other clinical-diagnostical determinations : search in situ of Thricomonas vaginalis or manilia, Collins' toluidine blue test, smell test (with KOH), nitrite test.

The probe is made of plastic transparent material, preferably polyethylene or PVC (medical grade).

The probe is 16 - 20 cm long and presents an external diameter of 4 - 6 mm.; the preferred diameters of the probe are (internal diameter x external diameter) 2,7 x 4 or 3,2 x 4,7 or 3,8 x 5,4 or 4 x 6 mm. Also other diameters are possible. The plastic material of the probe has a shore value of 94-87.

The indicator is applied inside the pipe with a mechanical injector through automatic operations and in a predetermined dosage.

Typically the indicator is applied to the pipe in the form of a liquid solution in a suitable solvent which is then lyophilized so to cause the adhesion of the solid indicator to the internal wall of the pipe.

In other embodiments of the invention, the indicator is firstly absorbed on a non-woven felt or cotton wood or other absorption solid and small pieces of the so treated material are introduced in the pipe.

This type of embodiment allows the easy introduction of different indicators in the pipe with the consequent possibility of more chemical-diagnostic determinations by a single test.

For determining the vaginal pH the described probe is introduced in the vagina near to the posterior fornix, the vaginal liquid enters in the probe through the hole 2 and flows from the head to the opposite end; the indicator, when comes into contact with the liquid, reacts giving a colour corresponding to the pH of the liquid and is compared with a colorimetric scale.

After each measure the probe is discarded.

The probe according to the invention presents several advantages respect of the prior art, particularly:
- elimination of the risks connected with a possible local and /or systemic toxicity caused by the contact of the indicator with the vaginal mucosa;
- unexpensiveness of production;
- easiness of use;
- possibility of being used with suitable colorimetric or physical indicators according to the wanted chemical-diagnostic determinations;
- elimination of the risk of transmitting bacterical or mycotic infections from one patient to the other and also AIDS or hepatitis viruses.

## Claims

1. Disposable probe for the determination of vaginal pH and other indices, consisting of a pipe (1) with closed head (3), characterized in that an oval hole (2) is provided in the wall of said pipe (1) near to said head (3) and in that one or more pH indicators or other indicators are applied on the internal surface of said head (3) and/or on the internal cylindrical surface of said pipe (1).

2. Disposable probe according to claim 1, characterized in that said indicators are applied at appropriately spaced positions (4, 5, 6) along said pipe (1) and/or near the end (7) of said pipe (1).

3. Disposable probe according to claim 1, characterized in that said indicators are suitable for determining any pH values and particularly values from 3.5 to 8.

4. Disposable probe according to claim 1, characterized in that the major diameter of said oval hole (2) is comprised between 8 and 12 mm.

5. Disposable probe according to claim 1, characterized in that said oval hole (2) has a rim inclined from up to down and from outside to inside.

6. Disposable probe according to claim 1, characterized in that the length of said pipe (1) is comprised between 16 and 20 cm.

7. Disposable probe according to claim 1, characterized in that the external diameter of said pipe (1) is comprised between 4 and 6 mm and the internal diameter of said pipe (1) is comprised between 2,7 and 4 mm.

8. Disposable probe according to claim 7, characterized in that said pipe (1) has an external diameter of 4 mm and an internal diameter of 2,7 mm.

9. Disposable probe according to claim 7, characterized in that said pipe (1) has an external diameter of 4,7 mm and an internal diameter of 3,2 mm.

10. Disposable probe according to claim 7, characterized in that said pipe (1) has an external diameter of 6 mm and an internal diameter of 4 mm.

11. Disposable probe according to claim 1, characterized in that it is made of transparent, plastic material.

12. Disposable probe according to claim 11, characterized in that it is made of polyethylene or PVC.

## Patentansprüche

1. Einwegsonde zur Bestimmung des vaginalen pH-Wertes und anderer Bestimmungsgrößen, bestehend aus einem Rohr (1) mit geschlossenem Kopf (3), dadurch **gekennzeichnet,** daß in der Wand des Rohres (1) nahe dem Kopf (3) ein ovales Loch (2) vorgesehen ist und daß an der Innenfläche des Kopfes (3) und/oder an der zylindrischen Innenfläche des Rohres (1) ein oder mehrere pH-Indikatoren oder andere Indikatoren angebracht sind.

2. Einwegsonde nach Anspruch 1, dadurch gekennzeichnet, daß die Indikatoren in Stellungen (4, 5, 6) längs des Rohres (1) angebracht sind, die einen geeigneten Abstand voneinander haben und/oder nahe dem Ende (7) des Rohres (1).

3. Einwegsonde nach Anspruch 1, dadurch gekennzeichnet, daß die Indikatoren geeignet sind zur Bestimmung irgendwelcher pH-Werte und insbesondere von Werten zwischen 3,5 und 8.

4. Einwegsonde nach Anspruch 1, dadurch gekennzeichnet, daß der größere Durchmesser des ovalen Loches (2) zwischen 8 und 12 mm liegt.

5. Einwegsonde nach Anspruch 1, dadurch gekennzeichnet, daß das ovale Loch (2) einen Rand hat, der von oben nach unten und von außen nach innen geneigt ist.

6. Einwegsonde nach Anspruch 1, dadurch gekennzeichnet, daß die Länge des Rohres (1) zwischen 16 und 20 cm liegt.

7. Einwegsonde nach Anspruch 1, dadurch gekennzeichnet, daß der Außendurchmesser des Rohres (1) zwischen 4 und 6 mm liegt und daß der Innendurchmesser des Rohres (1) zwischen 2,7 und 4 mm liegt.

8. Einwegsonde nach Anspruch 7, dadurch gekennzeichnet, daß das Rohr (1) einen Außendurchmesser von 4 mm und einen Innendurchmesser von 2,7 mm hat.

9. Einwegsonde nach Anspruch 7, dadurch gekennzeichnet, daß das Rohr (1) einen Außendurchmesser von 4,7 mm und einen Innendurchmesser von 3,2 mm hat.

10. Einwegsonde nach Anspruch 7, dadurch gekennzeichnet, daß das Rohr (1) einen Außendurchmesser von 6 mm und einen Innendurchmesser von 4 mm hat.

11. Einwegsonde nach Anspruch 1, dadurch gekennzeichnet, daß sie aus einem transparenten Kunststoffmaterial besteht.

12. Einwegsonde nach Anspruch 11, dadurch gekennzeichnet, daß sie aus Polyethylen oder aus PVC besteht.

## Revendications

1. Sonde à usage unique, pour la détermination du pH vaginal et d'autres grandeurs, constituée d'un tuyau (1) à tête fermée (3), caractérisée en ce qu'un trou ovale (2) est ménagé dans la paroi dudit tuyau (1) à proximité de ladite tête (3) et en ce qu'un ou plusieurs indicateurs de pH ou autres indicateurs sont appliqués sur la surface interne de ladite tête (3) et/ou sur la surface cylindrique interne dudit tuyau (1).

2. Sonde à usage unique selon la revendication 1, caractérisée en ce que lesdits indicateurs sont appliqués en des endroits espacés à intervalles appropriés (4, 5, 6) le long dudit tuyau (1) et/ou à proximité de la tête (7) dudit tuyau (1).

3. Sonde à usage unique selon la revendication 1, caractérisée en ce que lesdits indicateurs conviennent pour déterminer n'importe quelle valeur de pH et, en particulier, des valeurs de 3,5 à 8.

4. Sonde à usage unique selon la revendication 1, caractérisée en ce que le grand diamètre dudit trou ovale (2) est compris entre 8 et 12 mm.

5. Sonde à usage unique selon la revendication 1, caractérisée en ce que ledit trou ovale (2) possède un rebord incliné du haut vers le bas et de l'extérieur vers l'intérieur.

6. Sonde à usage unique selon la revendication 1, caractérisée en ce que la longueur dudit tuyau (1) est comprise entre 16 et 20 cm.

7. Sonde à usage unique selon la revendication 1, caractérisée en ce que le diamètre extérieur dudit tuyau (1) est compris entre 4 et 6 mm et le diamètre intérieur dudit tuyau (1) est compris entre 2,7 et 4 mm.

8. Sonde à usage unique selon la revendication 7, caractérisée en ce que ledit tuyau (1) possède un diamètre extérieur de 4 mm et un diamètre intérieur de 2,7 mm.

9. Sonde à usage unique selon la revendication 7, caractérisée en ce que ledit tuyau (1) possède un diamètre extérieur de 4,7 mm et un diamètre intérieur de 3,2 mm.

10. Sonde à usage unique selon la revendication 7, caractérisée en ce que ledit tuyau (1) possède un diamètre extérieur de 6 mm et un diamètre intérieur de 4 mm.

11. Sonde à usage unique selon la revendication 1, caractérisée en ce qu'elle est faite de matière plastique transparente.

12. Sonde à usage unique selon la revendication 11, caractérisée en ce qu'elle est faite de polyéthylène ou de PVC.
